# EUROPÄISCHE PATENTANMELDUNG

(11) **EP 0 641 755 A1**
(43) Veröffentlichungstag der Anmeldung: **08.03.1995**
(21) Anmeldenummer: 94113209.4
(22) Anmeldetag: 24.08.1994
(51) Int. Cl.: C07B 45/00, C07B 45/04, C07C 381/04, C07C 313/04, C07C 315/00

(54) **Verfahren zur Hydrosulfinierung von Olefinen**

(30) Priorität: 04.09.1993 DE 4329932
(71) Anmelder: HOECHST AKTIENGESELLSCHAFT, D-65929 Frankfurt am Main (DE)
(72) Erfinder: Herwig, Jürgen Kurt-Walter, DCh., D-52072 Aachen (DE); Keim, Wilhelm, Prof. Dr., D-52074 Aachen (DE)

(57) **Zusammenfassung**

Verfahren zur Herstellung von Olefinen durch Verwendung eines Palladiumkatalysators bei einer Temperatur oberhalb der Ceiling-Temperatur des SO₂-Olefin-Copolymersystems zur Synthese von Sulfin- und Sulfonsäurederivaten.

## Beschreibung

Die Hydrosulfinierung, d.h. die Addition von Schwefeldioxid und Wasserstoff an Olefine ist eine bisher kaum beachtete Synthesemethode.

So beschreiben Crabtree und Mitarbeiter die Umsetzung von Olefinen, Wasserstoff und Schwefeldioxid unter starker UV-Bestrahlung mittels sehr toxischer Quecksilbersensibilisatoren. Dabei erhält man ein Produktgemisch, das mit Perameisensäure zu Sulfonsäuren oxidiert wird (R.H. Crabtree, R.R. Ferguson, J. Org. Chem., 56, 1991, 5503).

Katalytische Verfahren zur Addition von SO₂ und H₂ an Olefine zur einfachen Synthese von Sulfinsäuren, Sulfonsäuren und deren Derivaten sind nicht bekannt.

Aufgabe ist es daher ein katalytisches Verfahren zur Hydrosulfinierung von Olefinen zu entwickeln.

Gegenstand der Erfindung ist ein Verfahren zur Hydrosulfinierung von Olefinen, dadurch gekennzeichnet, daß die Hydrosulfinierung in Gegenwart von Palladiumkatalysatoren bei einer Temperatur oberhalb der Ceiling-Temperatur (T_{Ceil}) des SO₂-Olefin-Copolymersystems (J. Polymer Sci. 26 (1957) 351) bis zu einer Temperatur von 160°C erfolgt.

Für das erfindungsgemäße Verfahren geeignete Olefine sind C₃-C₁₈-Alkene, vorzugsweise C₃-C₈-Alkene, C₃-C₆-Alkenyl-C₆-C₁₀-aryle und C₅-C₈-Cycloalkene.
Die Alkene können verzweigt oder geradkettig, einfach oder mehrfach ungesättigt und/oder in α-Stellung mit einer Gruppe OR (R = C₁-C₆-Alkyl, C₆-C₁₀-Aryl) bzw. mit einer Gruppe wie COOR, COR oder CN, die zu einer Aktivierung der Doppelbindung führt, versehen sein. Nicht geeignet sind Alkene mit konjugierten Doppelbindungen.
Beispiele für einfach oder mehrfach ungesättigte Alkene sind Propen, Isobuten, n-Hexen, Dodecen, 1,9-Decadien und 1,5-Hexadien. Beispiele für Cycloalkene sind Cyclohexen und Cyclopenten. Als Alkenylaryl sei Allylbenzol beispielhaft erwähnt. Beispiele für Alkene mit aktivierten Doppelbindungen sind α-β-ungesättigte Carbonylverbindungen und Carbonsäureester, insbesondere Methylvinylketon und Acrylsäureester.

Besonders bevorzugte Olefine für das erfindungsgemäße Verfahren sind Propen, Buten und Isobuten.

Unter T_{Ceil} des SO₂-Olefin-Copolymersystems versteht man die Temperatur bei der sich Kettenwachstum und Kettenzerfall im Gleichgewicht befinden. Oberhalb T_{Ceil} findet keine Polymerisation statt, d.h. es liegen monomeres Olefin und SO₂ vor.
Die Hydrosulfinierung wird bevorzugt bei einer Temperatur von 80 bis 120°C durchgeführt.

Als Katalysator werden bevorzugt Pd(II)salze mit chelatisierenden Liganden wie Bis(di-C₆-C₁₀-arylphosphino)-C₁-C₆-alkyl und Bis(di-C₁-C₁₂-alkylphosphino)-C₁-C₆-alkyl verwendet. Besonders bevorzugt sind Bis(diphenylphosphino)-C₂-C₄-alkyl- und Bis(di-C₁-C₄-alkylphosphino)-C₂-C₄-alkylpalladium(II)salze.
Die Katalysatormenge beträgt 0,033 mmol/l-0,33 mol/l, vorzugsweise 0,333 mmol/l -0,033 mol/l.
Das Molverhältnis Olefin/SO₂ beträgt 1:10 - 10:1, vorzugsweise 1:5 - 5:1, insbesondere 1:1.
Das Molverhältnis Olefin/H₂ beträgt 1:10 - 10:1, vorzugsweise 1:2-1:3.

Zur Synthese eines geeigneten Katalysators werden äquimolare Mengen eines Pd(II)-Salzes wie PdCl₂ oder PdBr₂ und ein Ligand der Formel R₂P(CH₂)ₙPR₂ (R = Me, Et, Pr, i-Pr, Ph, p-Tol, 2-MeOPh, Cy, t-Bu, n = 1-6) in einem inerten Lösemittel (z.B. DMSO) solange gerührt, bis das gesamte Pd-Salz reagiert hat. Das Lösemittel wird abdestilliert und der gebildete Palladiumkomplex in quantitativer Ausbeute isoliert.
Der in einer geringen Menge inertem Lösungsmittel suspendierte Palladiumkomplex wird mit der zweifachen molaren Menge eines Salzes der Formel MX (X = nicht koordinierendes Anion, z.B.: BF₄⁻, B(C₆H₅)₄⁻, B(C₆F₅)₄⁻, PF₆⁻, SbF₆⁻, M =Ag, Tl), gelöst in einem schwach koordinierenden, polaren Lösemittel (z.B. Acetonitril, Dimethylformamid, Dimethylsulfoxid), versetzt. Nach vollständiger Reaktion wird das ausgefallene Salz (MCl oder MBr) abfiltriert.
Der Palladiumkatalysator kann isoliert oder direkt als Lösung eingesetzt werden.

Die Hydrosulfinierungsreaktion kann in einem Autoklaven mit Rührvorrichtung erfolgen, bevorzugt in Inertgasatmosphäre. Zunächst wird die Katalysatorlösung zugegeben. Anschließend wird das SO₂ und das Olefin eingefüllt, danach wird der Wasserstoff aufgepreßt. In welcher Reihenfolge Olefin und SO₂ eingefüllt werden hängt vom Siedepunkt des benutzten Olefins ab, d.h. Olefine mit kleinerem Partialdruck als SO₂ werden vor dem SO₂ aufgepreßt.

Flüssige Olefine (z.B. 1-Hexen) und andere flüssige Zusätze werden gleich nach der Katalysatorlösung zugegeben. Dann werden das SO₂ und der Wasserstoff aufgepreßt.
Feste Olefine und andere feste Zusätze werden in einem Lösemittel gelöst und in den Autoklaven gegeben. Danach werden die Gase aufgepreßt.
Nach dem Abkühlen werden die Autoklaven gewogen und der Enddruck abgelesen. Nun wird vorsichtig der Druck abgelassen und nach längerem Ausrühren der im Lösemittel gelösten Gase nochmals gewogen. Der Autoklav wird aufgeschraubt und die Reaktionsprodukte herausgenommen. Anschließend wird das Lösemittel entfernt und das verbleibende Produkt analysiert.

Die Isolation der reinen Verbindungen gelingt durch übliche Trenntechniken (Destillation, Extraktion, Chromatographie).

Unter den vorgenannten Bedingungen entstehen bei dem erfindungsgemäßen Verfahren Thiosulfonsäureester und Sulfonsäuren im molaren Verhältnis 1:1. Sulfinsäure und/oder Sulfinsäurederivate erhält man nach dem erfindungsgemäßen Verfahren, wenn man die Hydrosulfinierungsreaktion in Gegenwart eines Alkohols und/oder Wasser durchführt, vorausgesetzt, daß das Wasser mit dem eigentlichen Lösungsmittel mischbar ist.
Geeignete Alkohole sind C₁-C₆-Alkanole, vorzugsweise Methanol und Ethanol. Das molare Verhältnis von Katalysator zu Alkohol und/oder Wasser beträgt 1:100 - 1:5000, vorzugsweise 1:500 - 1:1000.
Wird bei der Hydrosulfinierungsreaktion als Olefin ein Alken mit aktivierter Doppelbindung eingesetzt, so bildet sich intermediär die entsprechende Sulfinsäure, die mit überschüssigem Olefin zu dem entsprechenden Sulfon weiterreagiert.

Werden bei der Hydrosulfinierungsreaktion ein 1-Alken und ein Alken mit aktivierter Doppelbindung eingesetzt, so reagiert die aus dem 1-Alken gebildete Sulfinsäure mit dem Alken mit aktivierter Doppelbindung zu dem entsprechenden γ-Ketosulfon.

Die nach dem erfindungsgemäßen Verfahren erhältlichen Sulfin- und/oder Sulfonsäurederivate finden Verwendung als Wirkstoffe oder Wirkstoffvorprodukte für Pflanzenschutzmittel und Pharmaka. Außerdem können sie als Zwischenprodukte für Reaktivfarbstoffe eingesetzt werden.

Die Synthese der in den Beispielen verwendeten Katalysatoren wird nachfolgend beschrieben:

### Darstellung von Palladium[1,3-bis(diphenylphosphino)propan](dichlorid)

Zur Herstellung von Palladium[1,3-bis(diphenylphosphino)propan](dichlorid), gibt man in DMSO, zu 0,98 g Palladiumchlorid (5,5 mmol) 2,25 g dppp (5,5 mmol) und erhitzt unter Rühren bis sich PdCl₂ vollständig gelöst hat. Beim Abkühlen fällt aus der gelb-orangen Lösung das grünliche Palladium[1,3-bis(diphenylphosphino)propan](dichlorid) aus. Die Ausbeute betrug 2,79 g = 86,4 % der Theorie.

### Beispiel 1

### Thiosulfonsäureester und Sulfonsäuren

0,01 mmol (1,3-Bis(diphenylphosphino)propan)palladiumchlorid werden in 30 ml CH₂Cl₂ gelöst und mit 0,02 mmol AgBF₄ gelöst in 0,5 ml Acetonitril, versetzt. Das nach 10-minütigem Rühren vollständig ausgefallene AgCl wird abfiltriert und die Lösung in einen 150 ml - Hastelloyautoklaven mit Magnetrührer überführt. Nachfolgend wird SO₂ bis zu einem Druck von 3,5 bar aufgepreßt, gefolgt von Propen bis zu einem Druck von 8 bar und Wasserstoff bis zu einem Druck von 25 bar. Der Autoklav wird in ein auf 100°C vorgeheiztes Ölbad überführt. Nach 17 h wird der Autoklav erst auf Raumtemperatur abgekühlt und dann entspannt. Vom Reaktionsgemisch wird das Lösemittel abdestilliert. Man erhält 2 g einer Mischung aus isomeren Propanthiosulfonsäureestern und Sulfonsäuren. Letztere werden durch Extraktion mit Wasser aus der rohen Mischung gewonnen und durch ¹H- und ¹³C-NMR-Spektroskopie charakterisiert. Die Thiosulfonsäureester werden entweder durch Destillation (Kp_{1.3}101°C) oder durch Säulenchromatographie über Silicagelsäulen (Lösemittel: Ethylacetat/Hexan 1:9) rein gewonnen und durch ¹H-¹³C-NMR-Spektroskopie charakterisiert. Das Verhältnis von linearen zu verzweigten Ketten kann durch gaschromatographische Analyse in Verbindung mit einer massenspektroskopischen Analyse bestimmt werden. Die Isomerenverteilung für die Propanthiosulfonsäurepropylester lautet: n/n 77,4%, n/i + i/n 20,3%, i/i 2,3%.

### Beispiel 2

### Isolierung der Sulfinsäuren

0,1 mmol (1,3-Bis(diphenylphosphino)propan)palladiumchlorid werden in 30 ml CH₂Cl₂ gelöst und mit 0,2 mmol AgBF₄ gelöst in 0,5 ml Acetonitril, versetzt. Das nach 10-minütigem Rühren vollständig ausgefallene AgCl wird abfiltriert und die Lösung in einen 150 ml - Hastelloyautoklaven mit Magnetrührer überführt. 2g Methanol werden in den Autoklaven gegeben und nachfolgend SO₂ bis zu einem Druck von 3,5 bar aufgepreßt, gefolgt von Propen bis zu einem Druck von 8 bar und Wasserstoff bis zu einem Druck von 25 bar. Der Autoklav wird in ein auf 100°C vorgeheiztes Ölbad überführt. Nach 17 h wird der Autoklav erst auf Raumtemperatur abgekühlt und dann entspannt. Vom Reaktionsgemisch werden Methylenchlorid und Methanol im Vakuum bei Raumtemperatur abdestilliert. Es werden 2,1 g rohe Reaktionsmischung erhalten, davon 80 Gew.% isomere Propansulfinsäuren und 20 Gew.% isomere Propansulfinsäuremethylester (¹H-NMR). Die Sulfinsäuren werden durch Extraktion mit Wasser rein erhalten. Die Mischung der Sulfinsäuren besteht, wie die Propansulfinsäureester auch, aus 85 Gew.% linearen und 15 Gew.% verzweigten Produkten.

### Beispiel 3

### Isolierung der Sulfone

0,1 mmol (1,3-Bis(diphenylphosphino)propan)palladiumchlorid werden in 30 ml CH₂Cl₂ gelöst und mit 0,2 mmol AgBF₄ gelöst in 0,5 ml Acetonitril, versetzt. Das nach 10-minütigem Rühren vollständig ausgefallene AgCl wird abfiltriert und die Lösung in einen 150 ml - Hastelloyautoklaven mit Magnetrührer überführt. 5 ml Methylvinylketon werden in den Autoklaven gegeben und nachfolgend SO₂ bis zu einem Druck von 3,5 bar aufgepreßt, gefolgt von Wasserstoff bis zu einem Druck von 25 bar. Der Autoklav wird in ein auf 80 °C vorgeheiztes Ölbad überführt. Nach 18 h wird der Autoklav erst auf Raumtemperatur abgekühlt und dann entspannt. Vom Reaktionsgemisch wird das Methylenchlorid abdestilliert. Man erhält 0.8 g Bis-(butan-3-on)-sulfon.(¹H-NMR (ppm): 2.25 (s, 3H), 3.02 (t, 7 Hz, 2H), 3.30 (t, 7Hz, 2H), ¹³C-NMR (ppm): 29.85, 35.32, 47.75, 203.86, IR(cm⁻¹): 1713.7, 1239.8, 1136.4).

### Beispiel 4

### Isolierung der γ-Ketosulfone

0,13 mmol (1,3-Bis(diphenylphosphino)propan)palladiumchlorid werden in 30 ml CH₂Cl₂ gelöst und mit 0,26 mmol AgBF₄ versetzt. Das nach 10-minütigen Rühren vollständig ausgefallene AgCl wird abfiltriert und die Lösung in einen 150 ml - Hastelloyautoklaven mit Magnetrührer überführt. 4 g Methylvinylketon werden in den Autoklaven gegeben und nachfolgend SO₂ bis zu einem Druck von 3,5 bar, Propen bis zu einem Druck von 8 bar und Wasserstoff bis zu einem Druck von 25 bar aufgepreßt. Der Autoklav wird in ein auf 80 °C vorgeheiztes Ölbad gegeben, nach 16,5 Stunden auf Raumtemperatur abgekühlt und entspannt. Vom Reaktionsprodukt wird das Methylenchlorid und das Acetonitril abdestilliert. Es werden 5,0 g 2-Butanon-4-(propylsulfonyl) erhalten. Das Produkt ist ein Gemisch aus 85 Gew. % linearem und 15 Gew. % verzweigtem Produkt.

## Patentansprüche

1. Verfahren zur Hydrosulfinierung von Olefinen, dadurch gekennzeichnet, daß die Hydrosulfinierung in Gegenwart von einem Palladiumkatalysator bei einer Temperatur oberhalb der Ceiling-Temperatur des SO₂-Olefin-Copolymersystems bis zu einer Temperatur von 160°C erfolgt.

2. Verfahren gemäß Anspruch 1, dadurch gekennzeichnet, daß die Hydrosulfinierung bei Anwesenheit eines Alkohols und/oder Wasser im molaren Verhältnis von 100:1 - 5000:1 in Bezug auf den Katalysator durchgeführt wird.

3. Verfahren gemäß Anspruch 1 oder 2, dadurch gekennzeichnet, daß als Katalysator ein Pd(II)salz mit chelatisierendem Liganden verwendet wird.

4. Verfahren gemäß einem oder mehreren der Ansprüche 1, 2 oder 3, dadurch gekennzeichnet, daß als Katalysator ein Bisdiarylphosphinoalkanpalladium(II)salz verwendet wird.

5. Verfahren gemäß einem oder mehreren der Ansprüche 1, 2 oder 3, dadurch gekennzeichnet, daß als Katalysator ein Bisdialkylphosphinoalkanpalladium(II)salz verwendet wird.
